(19) Europäisches Patentamt
European Patent Office
Office européen des brevets

(11) Veröffentlichungsnummer: **0 139 023**
**B1**

(12) # EUROPÄISCHE PATENTSCHRIFT

(45) Veröffentlichungstag der Patentschrift: **10.08.88**

(51) Int. Cl.⁴: **C 07 D 207/46**, C 07 D 239/54, C 07 D 403/12, A 61 K 31/505

(21) Anmeldenummer: **84901562.3**

(22) Anmeldetag: **07.03.84**

(86) Internationale Anmeldenummer: **PCT/SU 84/00013**

(87) Internationale Veröffentlichungsnummer: **WO 84/03510 (13.09.84 Gazette 84/22)**

(54) **5-Fluoruracilnitroxylderivate.**

(30) Priorität: **11.03.83 SU 3557801**

(43) Veröffentlichungstag der Anmeldung: **02.05.85 Patentblatt 85/18**

(45) Bekanntmachung des Hinweises auf die Patenterteilung: **10.08.88 Patentblatt 88/32**

(84) Benannte Vertragsstaaten: **BE CH DE FR GB LI SE**

(56) Entgegenhaltungen:
**DE-A-2 455 423**
**GB-A-1 168 391**
**SU-A-721 439**

**CHEMICAL ABSTRACTS, Band 97, Nr. 15, 11. Oktober 1982, Seite 5, Zusammenfassung 119982q, Columbus, Ohio, US; W.K. SUBCZYNSKI: "Application of nitroxide free radicals in cancer chemotherapy"**

(73) Patentinhaber: **INSTITUT KHIMICHESKOI FIZIKI AKADEMII NAUK SSSR, Noginsky r-on Chernogolovka, 142432, Moskovskaya obl. (SU)**

(72) Erfinder: **EMANUEL, Nikolai Markovich, ul. Kosygina, 6-44, Moscow, 117334 (SU)**
Erfinder: **ROZENBERG, Albina Nikolaevna, Shkolny bulvar, 7-97 Chernogolovka, Moskovskaya obl. 142432 (SU)**
Erfinder: **GOLUBEV, Valery Alexandrovich, Institutsky pr., 6-115 Chernogolovka, Moskovskaya obl., 142432 (SU)**
Erfinder: **BOGDANOV, Gennady Nikolaevich, ul. Pervaya, 19/1-11 Chernogolovka, Moskovskaya obl. 142432 (SU)**
Erfinder: **VASILIEVA, Ljubov Sergeevna, ul. Tsentralnaya, 4-9 Chernogolovka, Moskovskaya obl. 142432 (SU)**
Erfinder: **KONOVALOVA, Nina Petrovna, ul. Dmitria Ulyanova, 4-2-339, Moscow, 117333 (SU)**

(74) Vertreter: **von Füner, Alexander, Dr., Patentanwälte v. Füner, Ebbinghaus, Finck Mariahilfplatz 2 & 3, D-8000 München 90 (DE)**

EP 0 139 023 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung bezieht sich auf neue biologisch wirksame Verbindungen, nämlich Uracilderivate, die geschwulsthemmende Wirksamkeit aufweisen.

Es ist das 5-Fluoruracil bekant, das zur Zeit in der Onkologie bei inoperablen Formen des Magendarmkanal -, des Lungen- und Eierstockkarzinoms Verwendung findet (J. Amer. Chem. Soc., V. 79, No. 16, herausgegeben 1957: R. Dushinsky, E. Pleven, C. Heidelberg "The syntesis of 5-Fluoropyrimidines", p. 4559-4560).

Diese Verbindung besitzt jedoch eine hohe Toxizität und verletzt besonders stark die Funktion des Knochenmarks und des Magendarmkanals.

Ein anderes Analogon der erfindungsgemäßen Verbindung ist 1-(α -Furanidyl)-5-fluoruracil (Ftorafur®) (GB-PS 1168391).

Diese Verbindung ist wenig toxisch ($LD_{50}$ für Mäuse beträgt 750 mg/kg), besitzt eine recht hohe Wirksamkeit in bezug auf solide experimentelle Geschwülste: Sarkom 180, Harding-Passey-Melanom, Walker-Karzinosarkom, alveoläres Leberkarzinom. In der Therapie wird das Arzneimittel auf Grundlage von Ftorafur® bei Mamma-, Rektum-, Dickdarm- und Magenkarzinom verwendet. Jedoch hemmt dieses Arzneimittel die Blutbildung und ruft Komplikationen seitens des Magendarmkanals hervor. Außerdem bleiben sowohl beim Ftorafur® als auch beim Fluroruracil paramagnetische Eigenschaften aus.

Der Erfindung liegt die Aufgabe zugrunde, neue Verbindungen zu entwickeln, die eine geschwulsthemmende Wirkung, geringe Toxizität und paramagnetische Eigenschaften besitzen.

Diese Ausgabe wird dadurch gelöst, daß 5-Fluorura-cilnitroxylderivate der allgemeinen Formel

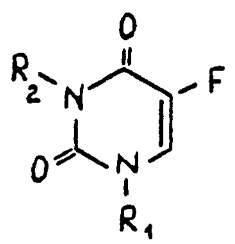

vorgeschlagen werden, worin bedeuten:

$R_1 = R_2 = X$,

X = 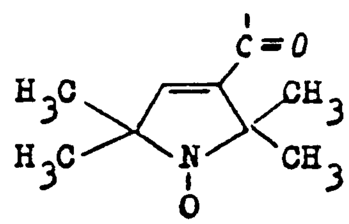

oder $R_1 = X$, $R_2 = H$,

oder $R_1 = H$, $R_2 = X$,

oder $R_1 = Na\oplus$, $R_2 = X$,

die eine geschwulsthemmende Wirksamkeit aufweisen.

Die genannten Verbindungen besitzen eine hohe geschwulsthemmende Wirksamkeit, die die zytostatische Wirksamkeit von Ftorafur® um das 2-4-fache übersteigt und mit der von 5-Fluoruracil verglichen werden kann.

Die erfindungsgemäßen Verbindungen besitzen eine geringere Toxizität, was gestattet, unerwünschte Nebenwirkungen herabzusetzen, die Efektivität der Hemmung der bösartigen Geschwulstneubildungen im Organismus zu erhöhen und das therapeutische Wirkungsspektrum bedeutend zu erweitern.

Das Vorliegen von parmagnetischen Eigenschaften bei den erfindungsgemäßen Verbindungen gestattet es, die Kontrolle der Therapie zu vereinfachen. Die Möglichkeit, paramagnetische Arzneimittel in den Geschwulstgeweben und die Stellen ihrer Lokalisation bis ans Zellenniveau zu bestimmen, macht diese Verbindungen bei der Untersuchung der Geschwulstprozesse und bei der Aufklärung des Wirkungsmechanismus der Zytostatika unentbehrlich.

Im weiteren wird die Beschreibung der Erfindung durch konkrete Beispiele der Ausführung der Erfindung und durch Zeichnungen veranschaulicht, worin:

Fig. 1 kinetische Kurven der Veränderung der Milzmasse von Mäusen bei La-Leukämie in der Kontrolle und bei der Einwirkung der äquimolaren Dosen von Ftorafur®, 5-Fluoruracil und 5-Fluoruracilnitroxylderivat ($R_1 = Na\oplus$, $R_2 = X$), die in den Koordinaten M- $\tau$ angegeben sind, worin bedeuten: M - Milzmasse der Mäuse in g, $\tau$ - Zeit in Tagen;

und Fig. 2 kinetische Kurven der Vergrößerung des Geschwulstvolumens des Adenokarzinoms 755 bei Mäusen in der Kontrolle und bei der Einwirkung der äquimolaren Dosen von Ftorafur® und 5-Fluoruracilnitroxylderivat ($R_1 = Na\oplus$, $R_2 = X$), die in den Koordinaten V- $\tau$ angegeben sind, worin bedeuten: V -

Geschwulstvolumen des Adenokarzinoms 755 in $cm^3$, $\tau$ - Zeit in Tagen, zeigt.

Die erfindungsgemäßen 5-Fluoruracilnitroxylderivate der genannten allgemeinen Formel, worin $R_1 = R_2 = X$ oder $R_1 = X$, $R_2 = H$ bedeuten, werden durch Kondensation des Bis-(trimethylsilyl)-5-fluoruracils (A) mit 2,2,5,5-Tetramethyl-1-oxyl- $\Delta^3$-pyrrolin-3 carbonsäurechlorid (B) im Medium eines inerten Lösungsmittels, beispielsweise Benzol, oder ohne Lösungsmittel bei Zimmertemperatur oder unter schwachem Erwärmen hergestellt. Bei einem Molverhältnis der Verbindung (A) zur Verbindung (B), das 1 : 2-3 beträgt, wird 1,3-Bis-(2,2,5,5-tetramethyl-1-oxyl- $\Delta^3$-pyrrolin-3-oyl)-5-fluoruracil ($R_1 = R_2 = X$) erhalten. Bei einem Molverhältnis der Verbindung (A) zur Verbindung (B), das 2-3 : 1 beträgt, wird 1-(2,2,5,5-Tetramethyl -1-oxyl- $\Delta^3$-pyrrolin-3-oyl)-5-fluoruracil ($R_1 = X$, $R_2 = H$) erhalten.

Die erfindungsgemässe Verbindung der allgemeinen Formel, worin $R_1 = Na\oplus$, $R_2 = X$ bedeuten, und zwar 3-(2,2,5,5-Tetramethyl-1-oxyl- $\Delta^3$-pyrrolin-3-oyl)-5-fluoruracilnatriumsalz wird durch die Behandlung der alkoholischen 1,3-Bis-(2,2,5,5-tetramethyl-1-oxyl- $\Delta^3$-pyrrolin -3-oyl)-5-fluoruracillösung mit der äquivalenten Natriumalkoholatmenge erhalten.

Die erfidungsgemässe Verbindung der allgemeinen Formel, worin $R_1 = H$, $R_2 = X$ bedeuten, und zwar 3-(2,2-5, 5-Tetramethyl-1-oxyl- $\Delta^3$-pyrrolin-3-oyl)-5-fluoruracil wird durch Behandlung des 3-(2,2,5,5-Tetramethyl-1-oxyl-$\Delta^3$-pyrrolin-3-oyl)-5-fluoruracilnatriumsalzes mit der äquivalenten Menge einer verdünnten Säure, beispielsweise der Salz- oder Schwefelsäure erhalten.

Weiter werden in den Text folgende Bezeichnungen der erfidungsgemässen Verbindungen eingeführt:
die Verbindung, worin $R_1 = R_2 = X$ bedeutet, wird als Verbindung (I);
die Verbindung, worin $R_1 = X$, $R_2 = H$ bedeuten, wird als Verbindung (II);
die Verbindung, worin $R_1 = H$, $R_2 = X$ bedeuten, wird als Verbindung (III);
die Verbindung, worin $R_1 = Na\oplus$, $R_2 = X$ bedeuten, wird als Verbindung (IV) bezeichnet.

Die Struktur der erfindungsgemässen 5-Fluoruracil-nitroxylderivate wird durch Elementeranalyse und durch Methoden der ESR-, IR-, Elektronen- und Massenspektrometrie bestätigt.

In den IR-Spektren der erfindungsemässen Verbindungen (I, II, III) liegt eine Gruppe von Banden in einem Bereich von 1670 bis 1760 $cm^{-1}$ vor, die durch Schwankungen der Carbonylgruppen des Uracils und der Pyrrolinoylsubstituenten bedingt worden sind.

In Übereinstimmung mit der Zahl der Carbonylgruppen enthalten die erfindungsgemäßen Verbindungen (II, III, IV) eine geringere Zahl von Carbonylbanden, als die Verbindung (I). Bei der Verbindung (IV) sind die Carbonylbanden des Uracils (1561 und 1673 $cm^{-1}$) zur Niederfrequenzseite infolge der Delokalisation der negativen Anionladung auf die Carbonylgruppen des Uracils verschoben.

In den IR-Spektren aller erfindungsgemäßen Verbindungen (I, II, III, IV) liegen auch die Banden der Valenzschwingungen der C=C-Bindungen des Pyrrolins und Uracils mit der Frequenz von 1620 bis 1625 $cm^{-1}$, die Banden der Valenzschwingungen der Protone der Pyrrolinmethylgruppen (2800-3000 $cm^{-1}$) und der H-C=C-Gruppen des Pyrrolins und Uracils (3030-3100 $cm^{-1}$) vor. Bei den erfindungemäßen Verbindungen (II, III) sind auch die Banden der NH-Gruppe mit der Frequenz von 3200 $cm^{-1}$ vorhanden.

Der Paramagnetismus der erfindungsgemäßen 5-Fluor-uracilnitroxylderivate wird durch die Methode der ESR bestätigt. Die Spektren der ESR der verdünnten Lösungen der Verbindungen (II, III, IV) bestehen aus drei Banden, die für Nitroxylmonoradikale kennzeichnend sind. Die ESR-Spektren der Verbindung (I) bestehen ans neun Banden und sind für die Nitroxylbiradikale kennzeichnend, bei denen die Energie der Elektronenaustauschkraft gleich 1,8 $aN^{14}$ ist. Die Konstanten der hyperfeinen Wechselwirkung $aN^{14}$ und die g-Faktoren der erfindungsgemäßen Verbindungen (I, II, III, IV) sind für die Nitroxylradikale der Pyrrolinreine kennzeichnend.

Die Elektronenspektren der Acetonitrillösungen der erfindungsgemäßen 5-Fluoruracilnitroxylderivate enthalten in einem Bereich von 12500 bis 50000 $cm^{-1}$ vier einander deckende Banden, die durch Absorption der Nitroxyl-, Pyrrolinoyl- und Uracilchromophore bedingt worden sind. Die Intensität dieser Banden bei der Verbindung (I) ist um das 1,5-2-fache höher, als bei den Verbindungen (II, III). Die Elektronenspektren der wäßrigen Lösungen der Verbindungen (II, III, IV) sind vom pH-Wert abhängig. Bei einem pH-Wert $\leqslant$ 3 sind diese hauptsächlich durch Absorption der Verbindungen (II oder III) und bei einem pH-Wert $\geqslant$ 11 - durch Absorption entsprechender Anionen bedingt.

Die Elektronenspektren der Verbindung (III) und des ihr entsprechenden Anions unterscheiden sich stark durch die Frequenz und Intensität der mit der Absorption des Uracilchromophors verbundenen Bande. Bei der obigen Verbindung hat diese Bande eine Frequenz von 41710 $cm^{-1}$, und beim Anion - 33710 $cm^{-1}$. Solch ein Unterschied in den Frequenzen ist für alle 3-substituierten Uracile kennzeichnend. Dadurch wird die Struktur der Verbindungen (III und IV) bestätigt. Die Spektren der Verbindung (II) und des ihr entsprechenden Anions sind einander nah, was für 1-substituierte Uracile auch kennzeichnend ist.

In den Massenspektren der Verbindungen (I, II, III) liegen Molekularionen M⊕ sowie Splitterionen vor, die die Zahl und die Stelle der Angliederung der X-Pyrrolinoylgruppen zum 5-Fluoruracil bestätigen, das als Fu bezeichnet wird. So liegen im Massenspektrum der Verbindung (III) die Splitterionen m/e 167 /X/⊕ und m/e 130 /Fu/⊕ sowie die Splitterionen m/e 226 /$XNHCONH_2$/⊕ und m/e 225 /XNHCONH/⊕ vor, die von der Angliederung der 2,2,5,5-Tetramethyl-1-oxyl- $\Delta^3$-pyrrolinoylgruppe zum Stickstoffatom des Uracils zeugen, das sich zwischen den Carbonylgruppen befindet. Im Massenspektrum der Verbindung (I) liegt das Splitterion m/e 296 /XFu/⊕ vor, daß durch Abspaltung einer der zwei X-Gruppen vom Molekularion M⊕ bedingt wird.

Zum Unterschied von 5-Fluoruracil sind die Verbindungen (I, II, III) in organischen Lösungsmitteln, Alkoholen, Aceton, Acetonitril, Dimethylformamid und Dimethylsulfoxid, gut löslich. Die Verbindung (IV) ist sehr gut

wasserlöslich. Deren wäßrige Lösungen haben eine alkalische Reaktion. Die Verbindungen (I, II, III) lassen sich in Wasser schlecht lösen. Jedoch sind die Verbindungen (II, III) in wäßrigen Alkalilösungen oder in Pufferlösungen bei einem pH-Wert $\geqslant$ 10 gut löslich.

Die Verbindungen (III, IV) sind gegen Hydrolyse recht beständig. Bei Zimmertemperatur weisen sie in einem pH-Bereich von 3 bis 11 keine merkliche hydrolytische Spaltung innerhalb von ca. 5 Stunden auf.

Die Verbindung (II) läßt sich in wäßrigen Alkalilösungen bis zum Fluoruracil und 2,2,5,5-Tetramethyl-1-oxyl-$\Delta^3$-pyrrolin-3-carbonsäure schnell hydrolysieren. Die Hydrolysegeschwindigkeit steigt mit der Erhöhung des ph-Wertes des Mediums an.

Nachstehend werden Beispiele zur Herstellung der 5-Fluoruracilnitroxylderivate sowie physikalische und spektroskopische Charakteristiken der so erhaltenen Verbindungen angeführt.

**Beispiel 1.**

Herstellung von 1,3-Bis-(2,2,5,5-tetramethyl-1-oxyl-$\Delta^3$-pyrrolin-3-oyl)-5-fluoruracil (Verbindung I).

Einer Lösung von 8, 11 g 2,2,5,5-Tetramethyl-1-oxyl-$\Delta^3$-pyrrolin-3-carbonsäurechlorid in 40 ml trockenen Benzol werden unter einer trockenen Stickstoffatmosphäre 5,49 g Bis-(trimethylsilyl)-5-fluouracil zugegeben. Die ausgefallenen orangefarbenen Kristalle der Verbindung (I) werden abfiltriert, mit einer geringen Menge von Benzol und Hexan gewaschen und getrocknet. Die Ausbeute beträgt 7,1 g /77 %, bezogen auf das eingesetzte Bis-(trimethylsilyl) -5-fluoruracil.

Die so erhaltene Verbindung wird aus Toluol umkristallisiert, dabei werden orangefarbene prismatische Kristalle von 195 bis 198° C Schmp. erhalten.

Gefunden in %: C 57,4 $\pm$ 0,3; H 5,9 $\pm$ 0,1; N 12,1 $\pm$ 0,1; F 3,7 $\pm$ 0,3; m/e 462 /M/⊕. $C_{22}H_{27}FN_4O_6$.

Berechnet in %: C 57,14; H 5,98; N 12,11; F 4,11.

Die Molekularmasse beträgt 462, 4745.

Das I-Spektrum in $CCl_4$, ν, $cm^{-1}$ (ε, 1/Mol.cm): 1621 (230) C=C; 1676 (620), 1705 (1100), 1729 (800), 1762 (520) C=O; 2870 (100), 2935 (220), 2984 (310) $CH_3$; 3030 (80), 3065 (70), 3095 (90) CH.

Das EPR-Spektrum in Toluol bei 25°C und der Konzentration der erhaltenen Verbindung von $5.10^{-4}$ Mol/l besteht aus 9 Linien mit einem Amplitudenverhältnis von 0,16 : 100 :29 : 33 : 95 : 32 : 24 : 86 : 0,15 und mit Abständen der Seitenlinien von der Zentrallinie von 0,41; 0,58; 1; 2,23. Der g-Faktor = 2,0056 $\pm$ $1.10^{-5}$; $aN^{14}$ = 14,18 $\pm$ 0,04 Gauss.

Das Elektronenspektrum in Acetonitril, $\nu_{max}$, $cm^{-1}$ (ε, 1/Mol.cm): 26000 $\pm$ 100 (81 $\pm$ 2), 37400 $\pm$ 100 (8200 $\pm$ 100), 44560 $\pm$ 60 (20900 $\pm$ 200), 47000 $\pm$ 100 (22400 $\pm$ 200).

Das Massenspektrum, m/e /Ion/⊕ ($I/I_{max}$, %): 463 /M+1/⊕ (1,4); 462 /M/⊕ (4,4); 447 /M-$CH_3$/⊕ (0,22); 432 /M-NO/⊕ (2,2); 296 /XFu/⊕ (5,7); 281 /X Fu-$CH_3$/⊕ (0,16); 266 /X Fu-NO/⊕ (4,7); 167 /X/⊕ (16); 152 /X-$CH_3$/⊕ (16); 139 /X-CO/⊕; 137 /X-NO/⊕ (52); 109 /X-NOCO/⊕ (100), worin X und Fu die oben angegebenen Bedeutungen haben.

**Beispiel 2.**

Herstellung von 1-(2,2,5,5-Tetramethyl-1-oxyl-$\Delta^3$-pyrrolin-3-oyl)-5-fluoruracil (Verbindung II).

Einer Lösung von 2,02 g 2,2,5,5-Tetramethyl-1-oxyl-$\Delta^3$-pyrrolin-3-carbonsäurechlorid in 100 al trockenem Benzol werden unter einer trockenen Stickstoffatmosphäre 5,49 g Bis-(trimethylsilyl)-5-fluoruracil zugesetzt. Die orangefarbig-roten Kristalle der Verbindung (II) werden abfiltriert, mit Benzol, dann mit Hexan gewaschen und getrocknet. Die Ausbeute beträgt 1,5 g (50 %, bezogen auf 2,2,5,5-Tetramethyl-1-oxyl-$\Delta^3$-pyrrolin-3-carbonsäurechlorid).

Die so erhaltene Verbindung wird aus Nitromethan umkristallisiert. Dabei werden orangefarbig-rote nadelförmige Kristalle von 198 bis 201° C Schmp. erhalten.

Gefunden in %: C 52,4 $\pm$ 0,3; H 5,10 $\pm$ 0,05; N 13,8 $\pm$ 0,2; m/e 296 /M/⊕. $C_{13}H_{15}FN_3O_4$.

Berechnet in %: C 52,70; H 5,10; N 14,18.

Die Molekularmasse beträgt 296, 2760.

Das IR-Spektrum in Acetonitril, ν, $cm^{-1}$ (ε, 1/Mol.cm): 1627 (110) C=C; 1685 (330), 1730 (1700) C=O; 2835 (40), 2872 (52), 2946 (155), 2983 (210) $CH_3$; 3105 (140) CH; 3225 (130) NH.

Das EPR-Spektrum in Toluol bei 25°C und der Konzentration der erhaltenen Verbindung von $5.10^{-4}$ Mol/l besteht aus 3 Linien mit dem Amplitudenverhältnis von 100 : 99 : 90; g=2,00562 $\pm$ $3.10^{-5}$; $aN^{14}$=14,25 $\pm$ 0,03 Gauss.

Das Elektronenspektrum in Acetonitril, $\nu_{max}$, $cm^{-1}$ (ε, 1/Mol.cm): 26120 $\pm$ 50 (50 $\pm$ 1), 37120 $\pm$ 50 (7700 $\pm$ 100), 40700 $\pm$ 40 (10000 $\pm$ 100), 47030 $\pm$ 40 (13400 $\pm$ 200).

Das Elektronenspektrum in 0,001 molarer wäßriger Salzsäurelösung, $\nu_{max}$, $cm^{-1}$ (ε, 1/Mol.cm): 41840 $\pm$ 80 (9700), 47320 $\pm$ 80 ($\leqslant$ 14400).

**Beispiel 3.**

Herstellung von 3-(2,2,5,5-Tetramethyl-1-oxyl-$\Delta^3$-pyrrolin-3-oyl)-5-fluoruracilnatriumsalz (Verbindung IV).
Zu 4,62 g 1,3-Bis(2,2,5,5-tetramethyl-1-oxyl-$\Delta^3$-pyrrolin-3-oyl)-5-fluoruracil (Verbindung I) gießt man 10 ml 1 molare Natriumethylatlösung in absolutem Ethanol zu.
Das Reaktionsgemisch wird vermischt, danach werden 40 ml Diethylether zugegeben. Der ausgefallene gelbe Salzniederschlag wird abfiltriert, mit Diethylether gewaschen und getrocknet. Die Ausbeute beträgt 3,05 g (96 %). Das Salz weist keinen deutlichen Schmelzpunkt auf, zersetzt sich allmählich bei 250-330° C.
Gefunden in %: N 13,0 ± 0,2. $C_{13}H_{14}FN_3O_4Na$.
Berechnet in %: N 13,20.
Das IR-Spektrum im Vaselinöl, ν, $cm^{-1}$: 1620 C=C; 1561, 1673, 1738 C=O; 3070 CH.
Das Elektronenspektrum in 0,001 molarer wäßriger Salzsäurelösung, $\nu_{max}$, $cm^{-1}$ (ε, 1/Mol.cm): 37400 ± 50 (7200 ± 100), 41700 ± 40 (13800 ± 100), 46700 ± 100 (12100 ± 100).
Das Elektronenspektrum in 0,001 molarer wäßriger Natriumhydroxidlösung, $\nu_{max}$, $cm^{-1}$ (ε, 1/Mol.cm): 33700 ± 30 (8100 ± 100), 44270 ± 50 (15700 ± 300).
Das EPR-Spektrum in Wasser bei 25°C und der Konzentration des so erhaltenen Salzes von $5.10^{-4}$ Mol/l besteht aus 3 Linien mit dem Amplitudenverhältnis von 100 : 101 : 80; g = 2,00514 ± $1.10^{-5}$, $aN^{14}$ = 15,88 ± 0,05 Gauss.

**Beispiel 4.**

Herstellung von 3-(2,2,5,5-Tetramethyl-1-oxyl-$\Delta^3$-pyrrolin-3-oyl)-5-fluoruracil (Verbindung III).
Zu 1,59 g 3-(2,2,5,5-Tetramethyl-1-oxyl-$\Delta^3$-pyrrolin-3-oyl)-5-fluoruracilnatriumsalz (Verbindung IV) gießt man 25 ml wäßrige 0,2 n-Salzsäurelösung zu. Das Gemisch wird innerhalb von 10 Minuten durchgemischt und mehrmals mit Chloroform extrahiert. Die Chloroformextrakte werden mit $Na_2SO_4$ getrocknet, das Lösungsmittel wird untar Vakuum verdampft und der Rückstand wird aus Toluol umkristallisiert. Man erhält so eine Verbindung in Form von kleinen gelben Nadeln von 169 bis 171°C Schmp. Die Ausbeute beträgt 1,11 g (75 %).
Gefunden in %: C 52,6 ± 0,2; H 5,09 ± 0,05; N 14,3 ± 0,1; F 6,44 ± 0,05; m/e 296 /M/⊕. $C_{13}H_{15}FN_3O_4$.
Berechnet in %: C 52,70; H 5,10; N 14,18; F 6,41.
Die Molekularmasse beträgt 296, 276.
Das IR-Spektrum in Acetonitril, ν, $cm^{-1}$ (ε, 1/Mol.cm): 1622 (180) C=C; 1680 (1330), 1723 (760), 1754 (960) C=O; 2877 (47), 2940 (130), 2989 (190) $CH_3$; 3087 (90) CH; 3270 (140) NH.
Das EPR-Spektrum in Toluol bei 25°C und der Konzentration der so erhaltenen Verbindung von $5.10^{-4}$ Mol/l besteht aus 3 Linien aus dem Amplitudenverhältnis von 100 : 99 : 90; g=2,00561 ± $1 \cdot 10^{-5}$; $aN^{14}$=14,15 ± 0,01 Gauss.
Das Elektronenspektrum in Acetonitril, $\nu_{max}$, $cm^{-1}$ (ε, 1/Mol.cm): 25140 ± 20 (52,6 ± 0,5), 38190 ± 20 (8500 ± 200), 42460 ± 20 (14000 ± 500), 47430 ± 60 (13200 ± 600).
Das Elektronenspektrum in 0,001 molarer wäßriger Salzsäurelösung, $\nu_{max}$, $cm^{-1}$ (ε, 1/Mol.cm): 37410 ± 60 (7200 ± 100), 41710 ± 30 (13900 ± 100), 46700 ± 70 (12000 ± 100).
Das Elektronenspektrum in 0,001 molarer wäßriger Natriumhydroxidlösung, $\nu_{max}$, $cm^{-1}$ (ε, 1/Mol.cm): 33710 ± 30 (8160 ± 150), 44270 ± 90 (15700 ± 300).
Das Massenspektrum, m/e /Ion/⊕ ($I/I_{max}$, %): 297 /M+1/⊕ (7,1); 296 /M/⊕ (23); 282 /HM-$CH_3$/⊕ (26); 281 /M-$CH_3$/⊕ (1,2); 266 /M-NO/⊕ (12); 226 /XNH CO $NH_2$/⊕ (0,44); 225 /XNH CONH/⊕ (1,4); 167 /X/⊕ (42); 152 /X-$CH_3$/⊕ (67); 139 /X-CO/⊕ (16); 137 /X-NO/⊕ (82); 136 /X-HNO/⊕ (100); 130 /Fu/⊕ (91); 109 /X-NOCO/⊕ (96), worin X und Fu die oben angegebenen Bedeutungen haben.
Es wurden biologische Untersuchungen der erfindungsgemäßen 5-Fluoruracilnitroxylderivate im Vergleich zu 5-Fluoruracil und Ftorafur® durchgeführt. Die Ergebnisse dieser Untersuchungen sind in der Tabelle angeführt.
Die Toxizität wurde an weißen rasselosen Mäusen nach intraperitonealer Verabreichung der Verbindungen bestimmt und nach der Methode von Behrens beurteilt (aufgrund der Kurven der Häufigkeit des Todes der Tiere je nach der Dosis).
Als Charakteristik für die Toxizität wurde die Dosis verwendet, die den Tod von 50 % der Versuchstiere hervorruft ($LD_{50}$).
Zur Bestimmung der Toxizität von 1,3-Bis(2,2,5,5-tetramethyl-1-oxyl-$\Delta^3$-pyrrolin-3-oyl)-5-fluoruracil (Verbindung I), 1-(2,2,5,5-Tetramethyl-1-oxyl-$\Delta^3$-pyrrolin -3-oyl)-5-fluoruracil (Verbindung II) und 3-(2,2-5,5-Tetramethyl-1-oxyl-$\Delta^3$-pyrrolin-3-oyl)-5-fluoruracil (Verbindung III) wurden diese intraperitoneal in Form einer Emulsion in 10 %-iger wäßriger Polyoxyäthylsorbitanmonooleatlösung eingeführt (Tween-80®, Ferak Berlin-West).
Zur Bestimmung der Toxizität von 3-(2,2,5,5-Tetramethyl -1-oxyl-$\Delta^3$-pyrrolin-3-oyl)-5-fluoruracilnatrium-salz (Verbindung IV), Ftorafur® und 5-Fluoruracil wurden diese auch intraperitoneal in Form von wäßrigen Lösungen eingeführt.
Die Untersuchungsergebnisse haben ergeben, daß Verbindung (I) um das 3,5-fache weniger toxisch ist, als 5-Fluoruracil. Die Toxizität der Verbindung (II) fällt praktisch mit der von 5-Fluoruracil zusammen. Die

Verbindungen (III und IV) sind 5mal weniger toxisch als 5-Fluoruracil und sind der Toxizität nach dem Ftorafur® nahe.

Bei der Bestimmung der zytostatischen Wirksamkeit wurden die Verbindungen (I, II, III, IV), 5-Fluoruracil und Ftorafur® in denselben Lösungsmitteln wie bei der Bestimmung der Toxizität verwendet.

Die Bestimmung der geschwulsthemmenden Wirksamkeit erfolgte an Transplantationstumoren Leukämie L-1210, Leukämie P-388, Leukämie La, Melanom B-16, Adenokarzinom 755 (Ca-755) von Mäusen sowie an Transplantationstumoren Erythromyelose von Shvets, Walker-Karzinosarkom (WKS), Guerin-Karzinom von Ratten.

Der Leukämietumor L-1210 wurde intraperitoneal in einer Menge von $10^5$ Zellen in 0,2 ml Aszitesflüssigkeit transplantiert. Die Verbindung (IV) wurde auch intraperitoneal täglich verabreicht, angefangen einen Tag nach der Tumortransplantation. Das Behandlungsschema ist in der Tabelle angeführt.

Der Leukämietumor P-388 wurde intraperitoneal transplantiert, es wurden $10^6$ Zellen in 0,2 ml Aszitesflüssigkeit transplantiert. Die Verbindung (IV) wurde auch intraperitoneal täglich verabreicht, angefangen einen Tag nach der Tumortransplantation. Das Behandlungsschema ist in der Tabelle angeführt.

Der Leukämietumor La wurde intraperitoneal mit der Suspension der leukämischen Zellen in physiologischer Kochsalzlösung transplantiert. Das Inokulum enthielt $10^8$ leukämische Zellen. Die Verbindungen (I, II, III, IV), 5-Fluoruracil und Ftorafur® wurden auch intraperitoneal verabreicht, dabei wurden die Verbindungen zuerst 3 Stunden nach der Tumortransplantation und nachher täglich verabreicht. Das Behandlungsschema ist in der Tabelle angeführt.

Die soliden Geschwülste (Melanom B-16, Adenokarzinom 755, Eryhromyelose von Shvets, Walker-Karzinosarkom, Guerin-Karzinom) wurden mit der Geschwulstsuspension in physiologischer Kochsalzlösung (Verdännung 1 : 1 der Masse nach) transplantiert, die zu 0,3 ml jedem Tier subkutan verabreicht wurde. Die zu untersuchenden Verbindungen wurden intraperitoneal verabreicht, angefangen einen Tag nach der Geschwulsttransplantation im Falle der Transplantation des Melanoms B-16, des Adenokarzinoms 755, des Walker-Karzinosarkoms oder 7 Tage nach der Transplantation im Falle der Transplantation der Erythromyelose von Shvets und des Guerin-Karzinoms. Das Behandlungsschema ist in der Tabelle angeführt.

Bei der Bestimmung der geschwulsthemmenden Wirksamkeit wurden die zu untersuchenden Verbindungen in äquitoxischen Dosen verabreicht. Ftorafur® wurde in der optimalen Dosis verwendet, die 300 mg/kg beträgt.

Als Kriterium für die geschwulsthemmende Wirksamkeit bei Leukämien P-388, L-1210 und La diente die Verlängerung der durchschnittlichen Überlebensdauer (VÜD) der Versuchstiere im Vergleich zu den Kontrolltieren, die in Prozenten ausgedrückt wird und nach der Formel

$$VÜD = \frac{100 \cdot (L_{VG} - L_K}{L_K}$$

berechnet wird, worin bedeuten:

$L_{VG}$ - die Überlebensdauer der Tiere in der Versuchsgruppe;

$L_K$ - die Überlebensdauer der Tiere in der Kontrollgruppe ohne Verabreichung der zu untersuchenden Verbindungen.

Als Index der geschwulsthemmenden Wirksamkeit bei solidden Geschwülsten wird die Hemmung (H) der Vergrößerung des Geschwulstvolumens im Vergleich zur Kontrolle verwendet, die in Prozenten ausgedrückt wird und nach der Formel

$$H = \frac{100 (V_K - V_{VG})}{V_K}$$

berechnet wird, worin bedeuten:

$V_K$ - durchschnittliches Geschwulstvolumen in der Kontrollgruppe;

$V_{VG}$ - durchschnittliches Geschwulstvolumen in der Versuchsgruppe.

Im Falle der Leukämie La und des Adenokarzinoms 755 ist in der Tabelle noch ein Kriterium der geschwulsthemmenden Wirksamkeit, und zwar das kinetische Kriterium $\kappa = 1 - \varphi_{VG}/\varphi_K$ angeführt, worin $\varphi_{VG}$ und $\varphi_K$ - spezifische Geschwindigkeiten des Geschwulstwachstums im Versuch bzw. in der Kontrolle bedeuten.

Die vergleichende Beurteilung der therapeutischen Wirkungsbreite wird nach den chemotherapeutischen Indizes $I_{40}$, $I_{60}$ und $I_{100}$ durchgeführt:

$$I_{40} = \frac{LD_{10}}{ED_{40}},$$

$$I_{60} = \frac{LD_{10}}{ED_{60}},$$

$$I_{100} = \frac{LD_{10}}{ED_{100}},$$

worin bedeuten: $LD_{10}$ - die Dosis, die den Tod von 10 % der Tiere hervorruft, $ED_{40}$, $ED_{60}$, $ED_{100}$ - effektive Dosen, die die Vergrößerung der Überlebensdauer der Tiere um 40, 60 bzw. 100 % hervorrufen.

**Tabelle**

| Die zu untersuchende Verbindung | Tiere Stamm | Tiere Menge | Toxizität, $LD_{50}$, mg/kg | Geschwulst | Transplantationsstelle | Kontrolle, Überlebensdauer, Tage |
|---|---|---|---|---|---|---|
| 1 | 2 | 3 | 4 | 5 | 6 | 7 |
| I | Mäuse C57B1 | 20 | 350 | La | i/p | 6,5 |
| I | Mäuse $CBAF_1$ | 20 | - | B-16 | s/k | - |
| II | Mäuse C57Bl | 20 | 150 | La | i/p | 6,5 |
| III | Mäuse C57Bl | 20 | 550 | La | i/p | 6,5 |
| IV | Mäuse $BDF_1$ | 20 | 510 | L-1210 | i/p | 7,5 |
| IV | Mäuse $BDF_1$ | 20 | - | L-1210 | i/p | 7,5 |
| IV | Mäuse $BDF_1$ | 20 | - | P-388 | i/p | 10 |
| IV | Mäuse $BDF_1$ | 20 | - | P-388 | i/p | 10 |
| IV | Mäuse C57Bl | 20 | - | La | i/p | 6,5 |
| IV | Mäuse C57Bl | 20 | - | La | i/p | 6,5 |
| IV | Mäuse $CBAF_1$ | 20 | - | B-16 | s/k | - |
| IV | Mäuse C57Bl | 20 | - | Ca-755 | s/k | - |
| IV | Ratten s/l | 20 | - | WKS | s/k | - |
| IV | Ratten | 20 | - | Erythromyelose von | s/k | - |

| | | | | | | |
|---|---|---|---|---|---|---|
| | s/l | | | Shvets | | |
| IV | Ratten s/l | 20 | - | Guerin-Karzinom | s/k | - |
| 5-Fluor-uracil | Mäuse C57Bl | 20 | 100 | La | i/p | 6,5 |
| dasselbe | Mäuse C57Bl | 20 | - | La | i/p | 6,5 |
| dasselbe | Mäuse $CBAF_1$ | 20 | - | B-16 | s/k | - |
| dasselbe | Ratten s/l | 20 | - | Erythromyelose von Shvets | s/k | - |
| Ftorafur® | Mäuse C57Bl | 20 | 640 | La | i/p | - |
| dasselbe | Mäuse C57Bl | 20 | - | La | i/p | - |
| dasselbe | Mäuse $CBAF_1$ | 20 | - | B-16 | s/k | - |
| dasselbe | Ratten s/l | 20 | - | Erythromyelose von Shvets | s/k | - |

**Tabelle /Fortsetzung/**

| Die zu untersuchende Verbindung | Einzeldosis, mg/kg | Behandlungsschema, Tage nach der Geschwulsttransplantation | Geschwulsthemmende Wirksamkeit | | | |
|---|---|---|---|---|---|---|
| | | | α | Hemmung % | VÜD, % | der chemotherapeutische Index |
| 1 | 8 | 9 | 10 | 11 | 12 | 13 |
| I | 75 | 1-7 | 0,1 | - | 30 | $I_{40}=4,0$ |
| I | 75 | 1-10 | - | 15 | - | - |
| II | 75 | 1-7 | 0,5 | - | 45 | $I_{40}=2,0$ |
| III | 65 | 1-7 | 0,2 | - | 40 | $I_{40}=7,8$ |
| IV | 65 | 1-9 | - | - | 100 | - |
| IV | 75 | 1-7 | - | - | 60 | $I_{60}=5,6$ |
| IV | 75 | 1-7 | - | - | 90 | - |
| IV | 100 | 1,3,5,7,9 | - | - | 100 | $I_{100}=4,2$ |

| | | | | | | |
|---|---|---|---|---|---|---|
| IV | 75 | 1-7 | 0,7 | - | 80 | - |
| IV | 100 | 1,3,5,7,9 | - | - | 60 | $I_{60}=4,2$ |
| IV | 75 | 1-10 | - | 30 | - | - |
| IV | 65 | 1-10 | 0,3 | 70 | - | - |
| IV | 60 | 1-10 | - | 75 | - | - |
| IV | 75 | 7-14 | - | 45 | - | - |
| IV | 60 | 7-16 | - | 20 | - | - |
| 5-Fluor-uracil | 25 | 1-7 | - | - | 57 | $I_{60}=3,6$ |
| dasselbe | 35 | 1,3,5,7 | 0,67 | - | 57 | - |
| dasselbe | 25 | 1-10 | - | 0 | - | - |
| dasselbe | 25 | 7-14 | - | 15 | - | - |
| Ftorafur® | 45 | 1-6 | 0,17 | - | 15 | - |
| dasselbe | 300 | 1,5,9 | - | - | 60 | $I_{60}=2,0$ |
| dasselbe | 300 | 1,5,9 | - | 10 | - | - |
| dasselbe | 90 | 7-14 | - | 15 | - | - |

Anmerkungen zur Tabelle:
1) s/l - stammlos
2) i/p - intraperitoneal
3) s/k - subkutan
4) WKS - Walker-Karzinosarkom

Wie aus der Tabelle zu ersehen ist, besitzen alle erfindungsgemäßen Verbindungen eine deutlich ausgeprägte antileukämische Wirkung.

So ist die Verbindung (I) im Falle der Leukämie La bei mit Ftorafur® vergleichbaren Behandlungsschemen dem VÜD-Index nach zweimal effektiver als Ftorafur®, und die kinetischen Kriterien α beider Verbindungen sind dem Wert nach einander nah. Die Verbindung (I) ist nach ihrer therapeutischen Wirkung auf Leukämie La dem 5-Fluoruracil nahe.

Die Verbindung (II) ist bei Leukämie La dem VÜD-Index und dem kinetischen Kriterium α nach dreimal effektiver als Ftorafur® und kann den genannten Indizes nach mit 5-Fluoruracil verglichen werden.

Die Verbindung (III) ist bei Leukämie La dem VÜD-Index nach zweimal effektiver als Ftorafur®, und die kinetischen Kriterien α beider Verbindungen sind dem Wert nach einander nahe. Die Verbindung (III) ist nach ihrer therapeutischen Wirkung auf Leukämie La dem 5-Fluoruracil nahe.

Am wirksamsten ist die Verbindung (IV), die bei Leukämie La beim Vergleich in äquitoxischen Dosen und bei vergleichbaren Behandlungsschemen Forafur® der Wirkungseffektivität nach um das 5-fache übersteigt.

Die Wirkungseffektivität der Verbindung (IV) ist mit der Wirkung von 5-Fluoruracil vergleichbar.

Die Verbindung (IV) weist auch eine hohe geschwulsthemmende Wirksamkeit bei Leukämien L-1210 und P-388 auf.

Am wirksamsten von den erfindungsgemäßen Verbindungen in bezug auf solide Geschwülste ist die Verbindung (IV). Die Verbindung (IV) ist bei vergleichbaren Dosen und Behandlungsschemen 2-3-mal effektiver als Ftorafur® und 5-Fluoruracil bei der Wirkung auf Eryhromyelose von Shvets, dreimal effektiver als Ftorafur® bei der Wirkung auf Melanom B-16 (5-Fluoruracil weist keine Wirksamkeit in bezug auf Melanom B-16 auf). Die Verbindung (IV) besitzt eine bedeutende geschwulsthemmende Wirksamkeit in bezug auf Adenokarzinom 755 und Walker-Karzinosarkom.

Alle erfindungsgemäßen Verbindungen werden durch hohe Werte der chemotherapeutischen Indizes gekennzeichnet. So ist beispielsweise bei Leukämie La der chemotherapeutische index $I_{60}$ der Verbindung (IV) 2-mal höher im Vergleich zu $I_{60}$ von Ftorafur® und 1,2-mal höher im Vergleich zu 5-Fluoruracil.

Um die antitumorale Wirksamkeit und die kinetische Charakteristik der therapeutischen Effektivität der Verbindung (IV), von Ftorafur® und 5-Fluoruracil bei Leukämie La an den Tag zu legen, wurde eine Versuchsserie an Mäusen C57Bl durchgeführt. Die Leukämie La wurde intraperitoneal mit einer Suspension der

leukämischen Zellen in physiologischer Kochsalzlösung transplantiert. Das Inokulum enthielt $10^8$ leukämische Zellen. Die Verbindung (IV), Ftorafur® und 5-Fluoruracil wurden auch intraperitoneal verabreicht, dabei wurde die erste Verabreichung 3 Stunden nach der Transplantation der Geschwulst und nachher täglich innerhalb von 7 Tagen durchgeführt.

Es wurde die Veränderung der Masse (M) der Milz der Mäuse in g innerhalb von 14 Tagen ($\tau$) bestimmt. Nach den erhaltenen Angaben wurde in den Koordinaten M-$\tau$ ein in Fig. 1 angeführtes Diagramm erstellt.

In Fig. 1 sind kinetische Kurven der Veränderung der Masse der Milz der Mäuse bei Leukose La im Kontrollversuch (ohne Einführung der zu untersuchenden Verbindung) und bei der Wirkung der äquimolaren Einzeldosen (0,2 mMol/kg) der zu untersuchenden Verbindungen dargestellt:

die kinetische Kurve 1 - für den Kontrollversuch,
die kinetische Kurve 2 - bei der Wirkung von Ftorafur®,
die kinetische Kurve 3 - bei der Wirkung von 5-Fluoruracil,
die kinetische Kurve 4 - bei der Wirkung der Verbindung (IV).

Aus den Neigungen der kinetischen Kurven der Vergrößerung der Milzmasse folgt, daß die Verbindung (IV) das Wachstum der Leukämie La am effektivsten hemmt. Ihre Wirksamkeit ist etwas höher als die von 5-Fluoruracil und übersteigt um ein Vielfaches die Wirksamkeit von Ftorafur®.

Um die geschwulsthemmende Wirksamkeit und die kinetische Charakteristik der therapeutischen Effektivität der Verbindung (IV) und des Ftorafurs® bei Adenokarzinom 755 an den Tag zu legen, wurde eine Versuchsserie an Mäusen C57Bl durchgeführt. Adenokarzinom 755 wurde mit der Geschwulstsuspension in physiologischer Kochsalzlösung (Verdünnung 1 : 1 der Masse nach) transplantiert, die jeder Maus zu 0,3 ml subkutan verabreicht wurde. Die zu untersuchenden Verbindungen wurden intraperitoneal täglich innerhalb von 10 Tagen verabreicht, angefangen einen Tag nach der Geschwulsttransplantation.

Es wurde die Vergrößerung des Volumens (V) der Geschwulst des Adenokarzinoms 755 in $cm^3$ innerhalb von 28 Tagen ($\tau$) bestimmt. Nach den erhaltenen Angaben wurde in den Koordinaten V-$\tau$ ein in Fig. 2 angeführtes Diagramm erstellt.

In Fig. 2 sind kinetische Kurven der Volumenvergrößerung der Geschwulst des Adenokarzinoms 755 im Kontrollversuch (ohne Verabreichung der zu untersuchenden Verbindung) und bei der Wirkung der äquimolaren Einzeldosen (0,2 mMol/kg) der zu untersuchenden Verbindungen dargestellt:

die kinetische Kurve 5 - für den Kontrollversuch und bei der Wirkung von Ftorafur®,
die kinetische Kurve 6 - bei der Wirkung der Verbindung (IV).

Auf der kinetischen Kurve 5 sind die Versuchspunkte, die dem Kontrollversuch entsprechen, in Form von Kreisen, und die Versuchspunkte, die dem Versuch unter Ftorafur®-Verwendung entsprechen, in Form von Quadraten dargestellt.

Wie aus Fig. 2 zu ersehen ist, hemmt die Verbindung (IV) bedeutend die Entwicklung der Geschwulst, während Ftorafur® keine geschwulsthemmende Wirksamkeit aufweist.

Wie aus den angeführten Untersuchungsergebnissen ersichtlich ist, vereinigt die Verbindung (IV) die beste Eigenschaft von 5-Fluoruracil - dessen hohe antileukämische Wirksamkeit, und die besten Eigenschaften von Ftorafur®- dessen niedrige Toxizität und dessen hohe geschwulsthemmende Wirksamkeit in bezug auf solide Tumore.

Die erfindungsgemäßen 5-Fluoruracilnitroxylderivate können in der Biologie und Medizin Verwendung finden.

## Patentansprüche

1. 5-Fluroruracilnitroxylderivate der allgemeinen Formel

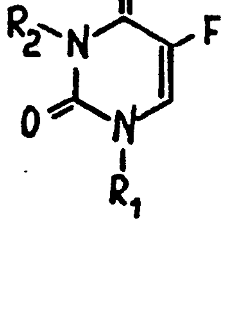

,

worin bedeuten: $R_1 = R_2 = X$

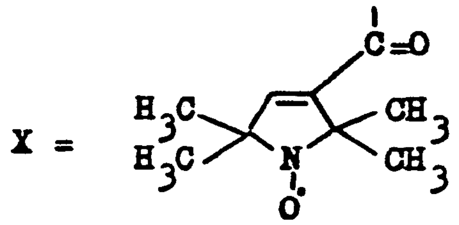

oder $R_1$ = X, $R_2$ = H,
oder $R_1$ = H, $R_2$ = X,
oder $R_1$ = Na⊕, $R_2$ = X,

2. Verfahren zur Herstellung von 5-Fluoruracilnitroxyl-derivaten nach Anspruch 1, dadurch gekennzeichnet, daß man Bis-(trimethylsilyl)-5-fluoruracil (A) mit 2,2,5,5-Tetramethyl-1-oxyl-$\Delta^3$-pyrrolin-3-carbonsäurechlorid (B) bei einem Molverhältnis von (A) zu (B) von 1 : 2 bis 3 oder 2 bis 3 : 1 kondensiert, wobei man 1,3-Bis-(2,2,5,5-tetramethyl-1-oxyl-$\Delta^3$-pyrrolin-3-oyl)-5-fluoruracil ($R_1 = R_2 = X$) oder 1-(2,2,5,5-Tetramethyl-1-oxyl-$\Delta^3$-pyrrolin-3-oyl)-5-fluoruracil ($R_1$=X, $R_2$=H) erhält, und die Verbindung nachfolgend mit einer äquivalenten Menge an Natriumalkoholat behandelt, wodurch man 3-(2,2,5,5-Tetramethyl-1-oxyl-$\Delta^3$-pyrrolin-3-oyl)-5-fluoruracilnatriumsalz erhält, das man durch Behandlung mit einer äquivalenten Menge einer anorganischen Säure in 3-(2,2,5,5-Tetramethyl-1-oxyl-$\Delta^3$-pyrrolin-3-oyl)-fluoruracil umwandelt.

3. Verwendung der 5-Fluoruracilnitroxylderivate nach Anspruch 1 zur Herstellung von geschwulsthemmenden Mitteln.

**Claims**

1. Nitroxyl derivatives of 5-fluorouracil of the general formula

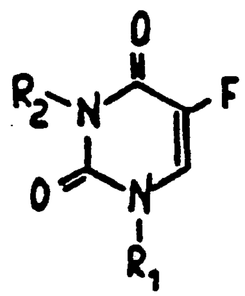

in which $R_1 = R_2 = X$

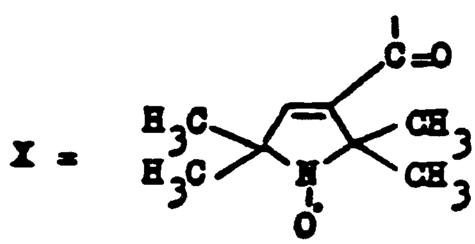

or $R_1$ = X, $R_2$ = H,
or $R_1$ = H, $R_2$ = X,
or $R_1$ = Na⊕, $R_2$ = X.

2. A process for the preparation of nitroxyl derivatives of 5-fluorouracil according to Claim 1, characterized in that bis-(trimethylsilyl-5-fluorouracil (A) is condensed with 2,2,5,5-tetramethyl-1-oxyl-$\Delta^3$-pyrroline-3-carboxylic acid chloride (B) with a molar ratio of (A) to (B) of from 1 : 2 to 3 or 2 to 3 : 1, in which case 1,3-bis-(2,2,5,5-tetramethyl-1-oxyl-$\Delta^3$-pyrroline-3-oyl)-5-fluorouracil ($R_1$ = $R_2$ = X) or 1-(2,2,5,5-tetramethyl-1-oxyl-$\Delta^3$-

pyrroline-3-oyl)-5-fluorouracil ($R_1$ = X, $R_2$ = H) is obtained, and the compound is subsequently treated with an equivalent quantity of sodium alcoholate, as a result of which 3-(2,2,5,5-tetramethyl-1-oxyl-$\Delta^3$-pyrroline -3-oyl)-5-fluorouracil sodium salt is obtained which is converted by treatment with an equivalent quantity of an inorganic acid to 3-(2,5,5-tetramethyl-1-oxyl-$\Delta^3$-pyrroline-3-oyl)-fluorouracil.

3. The use of the nitroxyl derivatives of 5-fluorouracil according to Claim 1 for the preparation of tumour-inhibiting agents.

**Revendications**

1. Dérivés de 5-fluorouracilnitroxyle de formule générale

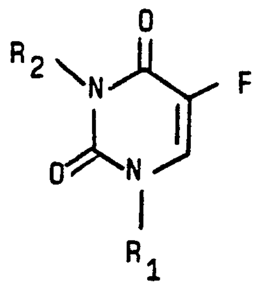

,

dans laquelle $R_1 = R_2 = X$

X =

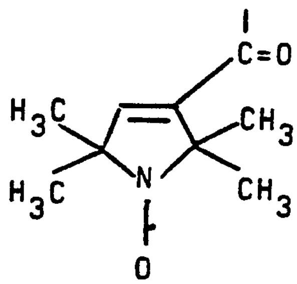

ou $R_1$ = X, $R_2$ = H,
ou $R_1$ = H, $R_2$ = X,
ou $R_1$ = Na⊕, $R_2$ = X.

2.- Procédé de préparation de dérivés de 5-fluorouracilnitroxyle selon la revendication 1, caractérisé en ca que l'on condense le bis-(triméthylsilyl)-5-fluorouracile (A) avec le chlorure de l'acide 2,2,5,5-tétraméthyl-1-oxyl-$\Delta^3$-pyrroline-3-carboxylique (B) avec un rapport molaire de (A) à (B) de 1 : 2 à 3 ou 2 à 3 : 1, obtenant ainsi le 1,3-bis-(2,2,5,5-tétraméthyl-1-oxyl-$\Delta^3$-pyrrolin-3-oyl)-5-fluorouracile ($R_1 = R_2 = X$) ou le 1-(2,2,5,5-tétraméthyl-1-oxyl-$\Delta^3$-pyrrolin-3-oyl)-5-fluorouracile ($R_1$=X, $R_2$=H), et en ce que l'on traite ensuite le composé avec une quantité équivalente d'alcoolate de sodium, obtenant ainsi le sel de sodium du 3-(2,2,5,5-tétraméthyl-1-oxyl-$\Delta^3$-pyrrolin-3-oyl)-5-fluorouracile, que l'on transforme en la 3-(2,2,5,5-tétraméthyl-1-oxyl-$\Delta^3$-pyrrolin-3-oyl)-fluorouracile par traitement avec une quantité équivalente d'un acide minéral.

3.- Utilisation des dérivés de 5-fluorouracilnitroxyle selon la revendication 1 pour la préparation d'agants antitumoraux.

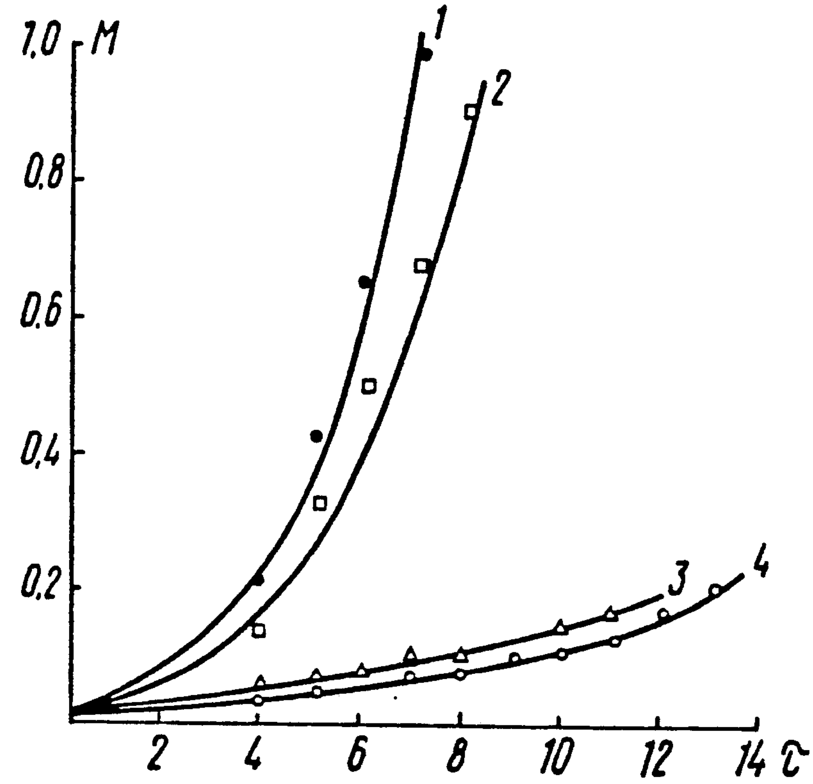


FIG. 1

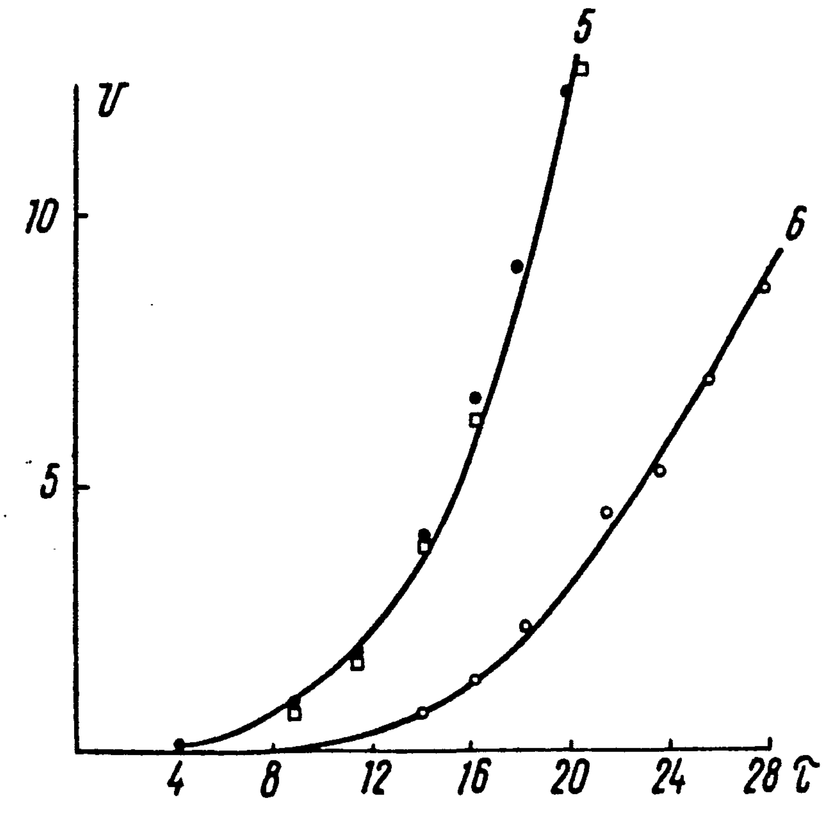


FIG. 2